# EUROPEAN PATENT APPLICATION

(11) **EP 4 825 309 A1**
(43) Date of publication of application: **21.10.2026**
(21) Application number: 25170520.8
(22) Date of filing: 15.04.2025
(51) Int. Cl.: A61B 5/00, A61B 5/145, A61B 5/1486

(54) **SEMI-INVASIVE CONTINUOUS ANALYTE MONITORING SYSTEM**

(71) Applicant: Sensirion AG, 8712 Stäfa (CH)
(72) Inventor: STREIFF, Matthias, 8032 Zürich (CH); STEINER-VANHA, Ralph, 8634 Hombrechtikon (CH); CAMINADA, Clara, 7000 Chur (CH)
(74) Representative: Schulz Junghans Patentanwälte PartGmbB

(57) **Abstract**

The present invention relates to a sensor unit (1) for use with a transceiver unit (2), comprising: a sensor (10) comprising a probe (11) configured to be placed under the skin to contact interstitial fluid and to measure a concentration of a first analyte in the interstitial fluid, wherein the sensor (10) is configured to provide an analog measurement signal indicative of the concentration of the first analyte, a signal processing integrated circuit (12) permanently electrically connected to the sensor (10), wherein the signal processing integrated circuit (12) is configured to receive the analog measurement signal from the sensor (10) and to e.g. convert it into a digital signal, a carrier (13) carrying the sensor (10) and the signal processing integrated circuit (12), and an optional releasable connection system (14) configured to releasably connect the sensor unit (1) to a transceiver unit (2).

## Description

The present invention relates to a sensor unit, particularly for a monitoring system for monitoring at least one analyte of a subject, e.g. a human or animal patient, or at least one analyte in a bioreactor.

In this regard, assemblies are known, where a sensor unit for measuring an analyte such as glucose is releasably connectable to a corresponding transceiver unit. However, such releasable assemblies often lead to a potentially unreliable electrical connection between the sensor unit and the transceiver unit through which nA range electrical currents are measured that suffer from contacts that are not perfectly clean. Particularly, the transceiver unit can be a reusable wearable device intended for prolonged contact with the user's skin. As such, it is exposed to environmental factors including perspiration, skin oils, dust, and other contaminants, which may affect its performance and durability over time, particularly its high-sensitivity electrical contacts. Furthermore, in traditional fixed-assembly type systems for analyte (e.g. glucose) monitoring, whenever the sensor unit needs changing (often at least every two weeks) the transceiver unit is discarded as well. This leads to unnecessary costs for the user and a waste of resources, especially given the scale at which such monitoring systems are used (~250 million units per year as of 2024).

Furthermore, future diagnostic assays require sensors capable of both multiplexed and multi-mode detection. For example, sensors may be designed to simultaneously measure multiple analytes, such as glucose and ketones, while also incorporating different detection modalities, including enzymatic, ion-selective, and immunological sensing technologies. Typically, four analog connections are available on the current devices, but six analog connections are needed for sensing two analytes such as glucose and insulin simultaneously when different modalities are concurrently measured that will result in a connector that will no longer fit in the housing of the product.

Based on the above, the problem underlying the present invention is to provide a sensor unit and a monitoring system comprising such a sensor unit that alleviate at least one of the above-mentioned difficulties.

This problem is solved by a sensor unit having the features of claim 1 and a monitoring system having the features of claim 13.

Preferred embodiments of these aspects of the present invention are stated in the corresponding dependent claims and are described below.

According to claim 1, a sensor unit for use with a transceiver unit is disclosed, comprising:
- a sensor comprising a probe configured to be placed under the skin of a human or animal subject to contact interstitial fluid and to measure a concentration of a first analyte in the interstitial fluid, particularly to provide continuous monitoring of the first analyte's concentration, wherein the sensor is configured to provide an analog measurement signal indicative of the concentration of the first analyte,
- a signal processing integrated circuit permanently electrically connected to the sensor in a non-detachable manner, wherein the signal processing integrated circuit is configured to receive the analog measurement signal from the sensor and to interact with the transceiver unit,
- a carrier carrying the sensor and the signal processing integrated circuit, and
- an optional releasable connection system configured to releasably connect the sensor unit to the transceiver unit.

Particularly, the signal processing integrated circuit and the sensor are permanently electrically connected to one another through direct electrical connections, without the use of any detachable electrical connector. This establishes a non-detachable connection, which leads to better reliability, lower cost, and higher miniaturization. Particularly, a non-detachable connection is a connection that cannot be released in a destruction-free manner, i.e., without cutting and/or heating the connection. Particularly, the non-detachable connection can be a soldered or a welded connection. Furthermore, in a preferred embodiment, the signal processing integrated circuit can be connected to a substrate (e.g. a flexible substrate, particularly a flexible printed circuit board, to which also the sensor may be connected or form a portion thereof, see below), using Au stud bumps with flip-chip assembly followed by soldering, using a wafer-level chip scale package (WLCSP) with solder bumps, or by wire bonding with subsequent glob top encapsulation.

Furthermore, particularly, the sensor unit may be used with a bioreactor instead of a subject for sensing one or several analytes contained in the bioreactor or guided towards or away from the bioreactor. In such an application, the probe is configured to contact the fluid to be analyzed, but does not necessarily be configured to be placed under the skin of a subject.

Furthermore, in a preferred embodiment, the signal processing integrated circuit is configured to process, particularly amplify, the analog measurement signal and/or to convert the analog measurement signal into a digital signal being indicative of the concentration of the first analyte, respectively.

Particularly, the signal processing integrated circuit has the properties to receive instructions and data from the transceiver unit to take measurements, interrogate one or more sensors, convert the analog measurement signal(s) to digital data, and return the digital data to the transceiver unit. This may be performed via electrical contacts in a wired fashion, or wirelessly, e.g., by a digital NFC connection that allows for a parallel power and data connection. Wired and wireless connections may also be combined, e.g. powering the sensor unit using two pins and carrying out the data transmission wirelessly by inductive or capacitive coupling. Optionally, the signal processing integrated circuit may also convert raw sensor data into calibrated data by calibration data stored on the signal processing integrated circuit and computing calibrated data from the raw data collected from the sensor(s). On this occasion, the signal processing integrated circuit may also combine data from different sensors to e.g. compensate one sensor signal with another, e.g. compensating a signal coming from an electrochemical sensor for temperature effects. Thus, particularly the unreliable high-pin-count analog electrical connection used in the prior art can be replaced by a digital connection (particularly including supply and ground connection (e.g. pin)) with e.g. two to four pins using e.g. a 1-wire or I2C interface. Or, if open contacts are to be avoided, by a digital NFC connection that can also power the sensor unit inductively at the same time.

The invention allows detaching the transceiver unit (preferably re-usable) from the sensor unit (preferably single use) with a reliable and durable wired or wireless electrical interface, hence reducing cost for the end user and unnecessary waste. Further, integrating the signal processing integrated circuit and the sensor on the same substrate using e.g. standard packaging processes further lowers the costs and low-cost and reliable sensor unit results.

Furthermore, the invention facilitates an arbitrary number of multiplex and combination of sensor modes (multi-mode) that may be routed through the same digital interface by serial communication, because the readout signal processing integrated circuit is now present on the sensor unit. Moreover, the signal processing integrated circuit now allows the sensor unit to provide a calibrated digital data output with calibration coefficients stored on the signal processing integrated circuit.

Particularly, according to a preferred embodiment, the probe of the sensor comprises a first working electrode (WE1) for an electrochemical reaction involving the first analyte (e.g. glucose or ketone), a reference Electrode (RE) for providing a stable potential reference, and a counter electrode (CE) for completing the circuit by allowing electrical current to flow.

In a preferred embodiment, the sensor's probe is configured to be placed under the skin to contact interstitial fluid and to measure a concentration of a first analyte and of different second analyte in the interstitial fluid particularly to provide continuous monitoring of the first and the second analyte's concentration, wherein the sensor is configured to provide analog measurement signals indicative of the concentrations of the first and the second analyte.

Particularly, in a preferred embodiment, to this end, the probe of the sensor comprises a first working electrode (WE1) for an electrochemical reaction involving the first analyte (e.g. glucose) and a second working electrode (WE2) for an electrochemical reaction involving the second analyte (e.g. ketone), wherein particularly the reference Electrode (RE) and the counter electrode (CE) can be shared with the first working electrode (WE1).

Particularly, the respective working electrode (WE1, WE2) is configured to be placed at the site of the enzymatic or non-enzymatic electrochemical reaction, where the respective (first or second) analyte (e.g. glucose or ketone) undergoes oxidation or reduction. Particularly, for measuring glucose as the first analyte, the first working electrode (WE1) can be coated with glucose oxidase (GOx), which catalyzes the oxidation of glucose to gluconolactone, generating a measurable electrical current. Further, particularly, for measuring ketones as the second analyte, the second working electrode (WE2) can be coated with β-hydroxybutyrate dehydrogenase, facilitating the oxidation of β-hydroxybutyrate (BHB) to acetoacetate, also generating a measurable electrical current. The amount of electrical current generated is proportional to the concentration of the respective (first or second analyte).

The reference electrode (RE) can comprise silver (Ag) coated with silver chloride (AgCl) to maintain a stable reference potential, wherein the electrochemical potential of the first or second working electrode is set relative to the reference electrode (RE). This ensures a consistent and controlled environment for the respective reaction. The counter electrode (CE) provides the return path for the generated electrical current to balances the electron flow at the respective working electrode by allowing reduction (or oxidation) reactions to occur. The counter electrode can be a platinum (Pt) or carbon electrode. A calibration curve that may be stored in the signal processing integrated circuit can be used to relate the measured electrical current (nA or µA) to the respective concentration of the first/second analyte (mg/dL or mmol/L).

Apart from ketones (e.g. β-Hydroxybutyrate) also analytes such as lactate and/or thrombin (T) can be measured instead of ketones or in addition thereto.

According to a preferred embodiment of the present invention, the sensor unit is a disposable sensor unit that is configured to be discarded after a predefined time span, wherein said time span is smaller than or equal to 3 weeks, particularly smaller than or equal to two weeks.

Particularly, the performance or functionality of the sensor unit can diminish after the defined time span, making reuse impractical or undesirable. Therefore, the sensor unit is designed for one-time use and is to be discarded after said predefined operational time span, beyond which its performance can be compromised. Particularly, the sensor unit is disposed of after the predefined time span to ensure accurate measurements and to prevent contamination or degradation that may affect subsequent readings.

Further, according to a preferred embodiment of the present invention, the signal processing integrated circuit is an ASIC (Application-Specific Integrated Circuit). Furthermore, in a preferred embodiment, the signal processing integrated circuit (e.g. ASIC) is e-beam sterilizable, i.e., it is capable of undergoing sterilization using high-energy electron beam irradiation without experiencing significant degradation in structural integrity, electrical performance, or functionality. Other common radiation based sterilization methods such as gamma and X-Ray may also be used depending on the susceptibility of the specific sensor material.

According to yet another preferred embodiment, the sensor unit comprises a substrate (e.g. a printed circuit board), wherein particularly the substrate is a flexible substrate (e.g. a flexible printed circuit board), wherein the signal processing integrated circuit is arranged on the substrate.

Furthermore, in a preferred embodiment, the sensor is one of: connected to the substrate, integrated into the signal processing integrated circuit or arranged thereon, arranged on the carrier.

Furthermore, in a preferred embodiment, the probe comprises an elongated filament configured to be placed under the skin to contact interstitial fluid, wherein the elongated filament is connected to the substrate or forms a portion of the substrate.

According to a preferred alternative embodiment, the probe comprises a microneedle array. Particularly, the microneedles in such an array can bear sensitive surfaces on their structure. Typically, each microneedle or area of microneedles carries one type, like a working electrode (WE), and is in direct contact with the interstitial fluid (ISF). This is similar to a filament, but the filament carries all types of electrodes, including working electrodes (WE), reference electrodes (RE), and counter electrodes (CE) on the same filament. Alternatively, the microneedle array can be configured to guide the fluid to be analyzed to the electrodes that may be arranged in a measuring chamber.

Furthermore, in a preferred embodiment of the invention, the signal processing integrated circuit is configured to calibrate the digital signal based on calibration data stored on the signal processing integrated circuit (e.g., ASIC).

Furthermore, according to a preferred embodiment, the sensor unit comprises an electrical connector configured to make an electrical connection to a corresponding electrical connector of the transceiver unit to provide electrical power to the sensor unit and/or to pass the (e.g. processed/amplified) analog measurement signal or the digital signal to the transceiver unit, and particularly to receive digital data from the transceiver unit.

Furthermore, according to yet another preferred embodiment, the sensor unit comprises an antenna, particularly a coil, configured for communication, particularly near field communication (NFC), with a corresponding antenna comprised by the transceiver unit to provide electrical power to the sensor unit and/or to transmit the digital signal(s) (or the analog measurement signal(s)) to the transceiver unit, and particularly to receive digital data from the transceiver unit.

Further, in a preferred embodiment of the invention, the releasable connection system comprises one of:
- a releasable mechanical connector configured to releasably engage with a mechanical connector of the transceiver unit to connect the sensor unit to the transceiver unit,
- an adhesive contact surface configured to contact the transceiver, providing a releasable adhesive connection of the sensor unit to the transceiver unit,
- a permanent magnet and/or a ferromagnetic element to facilitate a connection to the transceiver unit via a magnetic force.

According to a further preferred embodiment, the carrier is a housing (particularly a rigid housing) accommodating the signal processing integrated circuit, wherein the probe protrudes from the carrier (e.g. housing). In a preferred alternative embodiment, the carrier is a flexible patch, wherein the signal processing integrated circuit and the sensor are arranged on the flexible patch, wherein particularly the flexible patch is configured to be connected to a bottom side of a transceiver unit in form of a wristwatch (e.g. smartwatch), wherein particularly the wristwatch comprises at least one optical sensor (e.g. for heartrate measurement and/or for measuring peripheral capillary oxygen saturation, i.e., SpO2), wherein the flexible patch is configured to not cover the at least one optical sensor of the wristwatch when the flexible patch is connected to said bottom side.

According to a further aspect of the present invention, a monitoring system is disclosed, the monitoring system comprising a sensor unit according to the present invention and a transceiver unit, wherein particularly the transceiver unit comprises a transceiver circuit (e.g. Bluetooth Low Energy (BLE)) configured to communicate with a remote device, and a battery configured to supply the transceiver circuit (and particularly the sensor unit) with electrical power.

In a preferred embodiment of the monitoring system, the transceiver unit is a wristwatch, particularly a smartwatch, configured to be worn on the wrist of the subject (e.g. a human patient), wherein particularly the wristwatch has integrated therein the transceiver circuit and accommodates the battery.

According to a preferred embodiment of the monitoring system, the transceiver unit comprises an electrical connector configured to make an electrical connection to the electrical connector of the sensor unit to provide electrical power to the sensor unit and/or to receive the digital signal(s) (or the analog measurement signal(s)) from the sensor unit, and particularly to pass digital data to the sensor unit.

According to yet another preferred embodiment of the monitoring system, the transceiver unit comprises an antenna, particularly a coil, configured for communication, particularly near field communication (NFC), with an antenna of the sensor unit to provide electrical power to the sensor unit and/or to receive the digital signal(s) from the sensor unit, and particularly to transmit digital data to the sensor unit.

In the following exemplary embodiments as well as further features and advantages of the present invention are described with reference to the Figures. Particularly, each individual feature shown in the Figures and/or mentioned in the text describing the Figures may be incorporated (also in an isolated fashion) into a claim relating to the sensor device according to the present invention, wherein
- Figs. 1-2: show an embodiment of a sensor unit according to the present invention that can in particularly be used as a drop-in unit for existing analyte monitoring systems;
- Figs. 3-4: show an embodiment of a sensor unit and a monitoring system according to the present invention, wherein particularly the sensor unit is configured to communicate in a wireless fashion with a transceiver unit of the system;
- Fig. 5: shows a further embodiment of a sensor unit and a monitoring system according to the present invention, wherein particularly the sensor unit is configured to communicate with a transceiver unit of the system via an electrical connector; and
- Figs. 6-7: shows a further embodiment of a sensor unit and a monitoring system according to the present invention, wherein particularly the sensor unit is a patch (cf. Fig. 6B and 7) configured to be connected (cf. Fig. 6A) and communicate with a transceiver unit in form of a wristwatch.

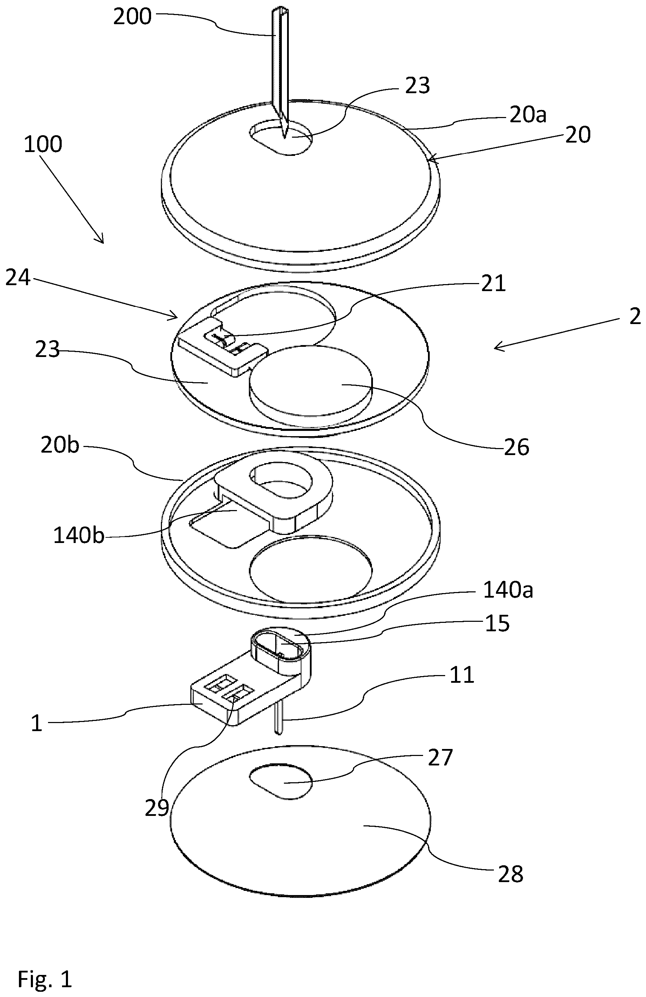

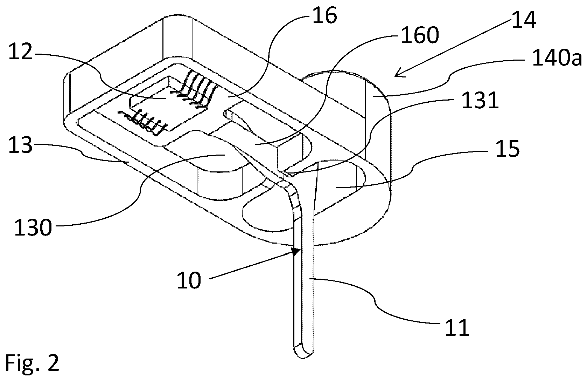

Fig. 2 shows in conjunction with Fig. 1 an embodiment of a sensor unit 1 according to the present invention. Particularly, the sensor unit 1 is configured to interact with a transceiver unit 2 as shown in Fig. 1. The sensor unit 1 comprises a sensor 10 comprising a probe 11 configured to be placed under the skin of a subject (e.g. a human or animal patient) to contact interstitial fluid and to measure a concentration of a first analyte (e.g. glucose), and particularly also of a second analyte (e.g. ketones), in the interstitial fluid, particularly to provide continuous monitoring of the respective analyte's concentration. Particularly, the sensor 10 is configured to provide an analog measurement signal indicative of the concentration of the first analyte, as well as a second measurement signal indicative of the concentration of the second analyte (in case the sensor 10 measures at least two analytes). Furthermore, the sensor unit 1 comprises a signal processing integrated circuit 12 permanently electrically connected to the sensor 10 in a non-detachable manner, wherein the signal processing integrated circuit 12 (e.g. an ASIC) is configured to receive the respective analog measurement signal from the sensor 10 and to convert it into a digital signal being indicative of the concentration of the respective (e.g. first or second) analyte.

The sensor unit 1 further comprises a carrier 13 in form of a housing which accommodates the sensor 10 and the signal processing integrated circuit 12, and a releasable connection system 14 configured to releasably connect the sensor unit 1 / housing 13 to the transceiver unit 2 (cf. e.g. Fig. 1). Particularly, the housing 13 may be connected to a housing 20 of the transceiver unit 2 in a releasable fashion, particularly by engaging with a protruding housing portion 140a in a recess 140b of the housing 20 of the transceiver unit. Particularly, the housing 13 of the sensor unit 1 comprises a through opening 15 for allowing passage of an introducer needle 200 by means of which the sensor probe 11 can be inserted under the skin. The through-opening 15 can be aligned with a corresponding opening 23 of the housing 20 of the transceiver unit 2 and a corresponding opening 27 of a patch 28 via which the monitoring system 100 comprising the sensor unit 1 and the transceiver unit 2 can be fixed to the skin of the subject. The aligned openings 23, 15 and 27 allow the passage of the introducer needle 200 through the transceiver and sensor units 1, 2, as well as through the patch 28.

As further indicated in Fig. 2, the signal processing integrated circuit 12 is arranged on a substrate 16, preferably in form of a flexible printed circuit board 16. Particularly, the probe 11 of the sensor is connected to the substrate 16 and can be integrally formed with the substrate 16. A main portion of the substrate 16 together with the signal processing integrated circuit 12 thereon can be accommodated in a recess 130 of the housing 13, wherein a connecting portion 160 of the substrate 160 that connects the probe 11 to said main portion extends through a further recess 131 of the housing 13 into the through-opening 15 of the housing 13. The further recess 131 particularly connects the recess 130 to the through-opening and forms a constriction. The connecting portion 160 can be twisted and can further be held and oriented by the further recess 131. Particularly, the connecting portion 160 can be inserted in the further recess 131 in a form-fitting manner allowing the elongated probe 11 to protrude out of the through-opening 11.

Furthermore, the probe 11 protrudes out of the through-opening 15 so that it can be inserted under the skin of the subject. Particularly, when the monitoring system 100 is attached to the skin as intended via patch 28, the elongated probe (e.g. filament) 11 extends through said opening 27 of the patch 28.

Furthermore, as shown in Fig. 1, the sensor unit 1 can be electrically connected to the transceiver unit 2 via an electrical connector 29 of the sensor unit 1 that can engage with a corresponding electrical connector 21 of the transceiver unit 2.

The electrical connector 21 of the transceiver unit 2 can be arranged on a substrate 23, particularly printed circuit board, of the transceiver unit 2, wherein the substrate 23 also accommodates a battery 26 of the transceiver unit 2 as well as a transceiver circuit 24 that allows the transceiver unit 2 to communicate with a remote device. Particularly, to transmit digital data to the remote device that can be indicative of the concentration of the respective (e.g. first or second) analyte. The substrate 23, the battery 26 and the transceiver circuit 24 arranged thereon are preferably housed in the housing 20 of the transceiver unit 2, which housing 20 can have a lower and an upper shell 20b, 20a.

The sensor unit 1 as shown in Fig. 2 can be configured to be used with existing systems as drop-in unit. Preferably, the sensor unit 1 is configured as a disposable sensor unit 1 that is configured to be discarded after a predefined time span, wherein said time span is smaller than or equal to 4 weeks, particularly smaller than or equal to three weeks, particularly smaller than or equal to two weeks In contrast thereto, the transceiver unit 2 is preferably configured to be a re-usable device and is also designed to supply the sensor unit 1 with electrical energy via the electrical connectors 29, 21.

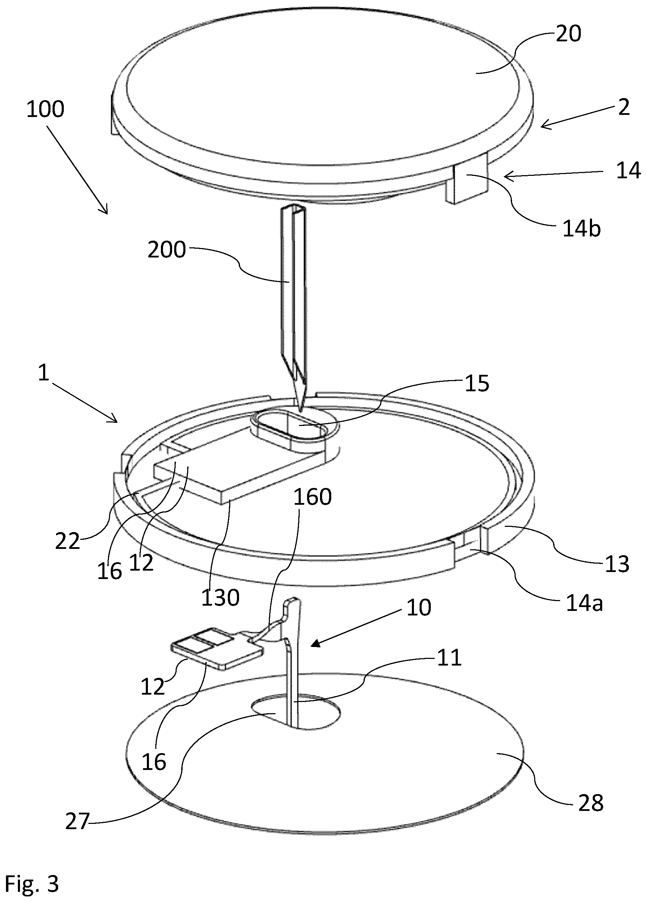

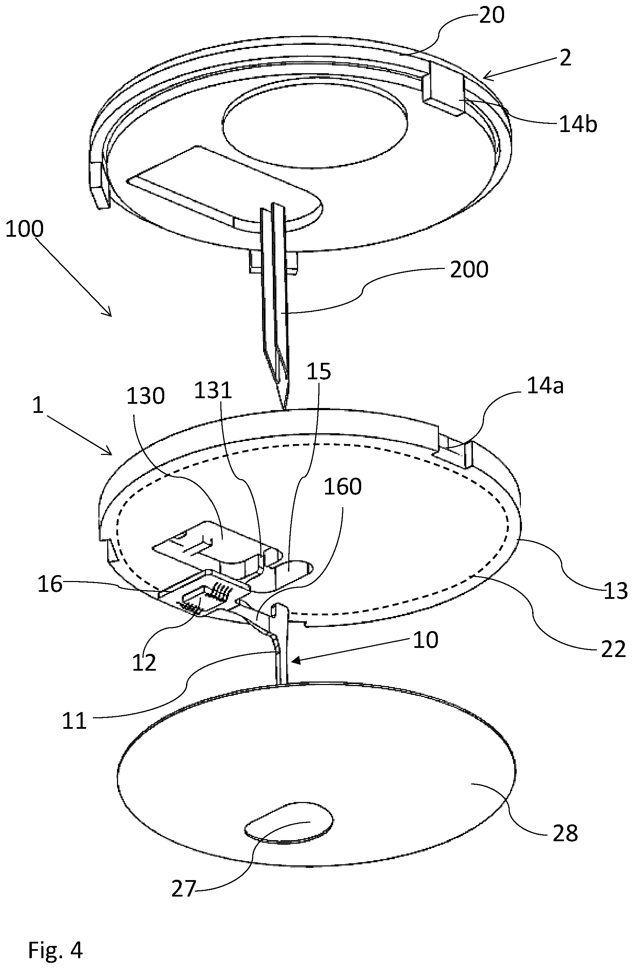

Furthermore, Fig. 3 shows in conjunction with Fig. 4 a further embodiment of a sensor unit 1 and a monitoring system for monitoring a concentration of an analyte sensed with the sensor unit 1 according to the present invention. The sensor unit 1 comprises a sensor 10 having a probe 11 facilitating measurement of a concentration of a first analyte (e.g. glucose) and particularly also of a second analyte (e.g. ketones) as described herein.

The sensor 10 is directly electrically connected to a signal processing integrated circuit (e.g. ASIC) 12 that is arranged on a substrate 16 (e.g. a flexible printed circuit board). Again, the sensor 10 comprises an elongated probe 11 that can be integrally connected to the substrate 16 as described in conjunction with Fig. 2. The sensor 10 provides analog measurement signals per analyte that are converted into digital signals by the signal processing integrated circuit 12.

The substrate 16 with the integrated circuit 16 thereon is accommodated in a recess 130 of the carrier 13 formed by an e.g. circular housing 13, wherein the sensor probe 11 extends out of a through-opening 15 formed in housing 13 to that the probe 11 can be inserted under the skin of a subject. The sensor unit 1 can communicate with a transceiver unit 2 via an antenna 22 that can e.g. establish NFC communication with the transceiver unit 2. Furthermore, electrical power for operating the sensor unit 1 may also be received from the sensor unit 1 via antenna 22 from the transceiver unit 2. The latter can comprise a battery and a transceiver circuit configured to transmit digital data received from the sensor unit 1 to a remote device. Particularly, the antenna 22 can be a coil 22 that extends along the periphery of the housing 13 as shown e.g. in Fig. 3.

The elongated probe 11 (e.g. filament) of the sensor 10 can be inserted under the skin of the subject by means of an introducer needle 200 that can be passed through the through-opening 15 of the housing 13. A housing 20 of the transceiver unit 2 that accommodates the components of the transceiver unit 2, such as the battery for powering the sensor unit and the transceiver circuit, is configured to be releasable connected to the housing 13 of the sensor unit 1 by means of a releasable connection system 14 that can comprises latching hooks 14b comprised by the housing 20 of the transceiver unit 2 that can engage with corresponding recesses 14a of the housing 13 of the sensor unit 1, which recesses 14a can comprise undercuts for engagement with the latching hooks 14b. This allows the sensor unit 1 to be a disposable unit while the transceiver unit 2 can be re-used as it comprises the power supply and the transceiver circuit. Alternatively, the sensor unit 1 may be releasably connected to the transceiver unit 2 based on an adhesive contact surface of e.g. the sensor unit 1 or by means of a permanent magnet and/or a ferromagnetic element to facilitate a connection to the transceiver unit via a magnetic force.

Furthermore, the sensor unit 1 and the transceiver unit 2 releasably connectable thereto may be attached to the skin of the subject by means of a patch 28 that comprises an opening 27 that can be aligned with the through-opening 15 of the housing 13 of the sensor unit 1 so that the probe 11 and introducer needle 200 can pass through the patch 28. The recess 130 and the through-opening 15 of the housing 13 may be connected as described before by a further recess through which a connecting portion 160 of the substrate 16 extends, which connecting portion 160 connects the probe 11 to a main portion of the substrate 16 on which the signal processing integrated circuit is 12 arranged. The elongated probe 11 (e.g. filament) protrudes out of the through-opening 15 of the housing 13 and can be inserted under the skin of the subject to measure the respective (e.g. first or second) analyte as described herein.

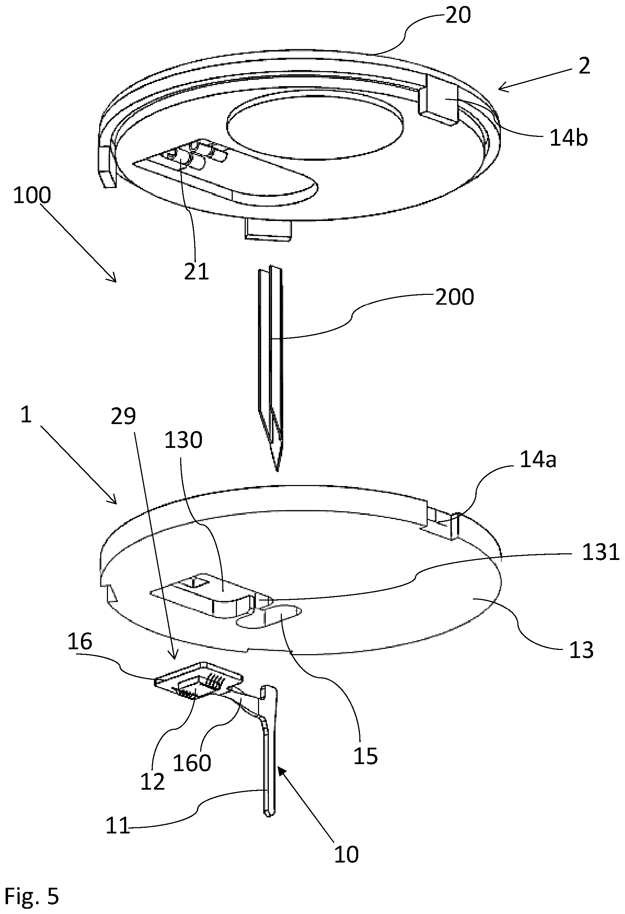

Fig. 5 shows a modification of the embodiment shown in Figs 3 to 4, wherein in contrast to Figs. 3 to 4, instead of an antenna (e.g. coil) 22, the sensor unit 1 of Fig. 5 comprises an electrical connector 29 configured to make an electrical connection to a corresponding electrical connector 21 of the transceiver unit 2 to provide electrical power to the sensor unit 1 and/or to pass the digital signal(s) indicative of the analyte concentration(s) to the transceiver unit 2, and particularly to receive digital data from the transceiver unit 2.

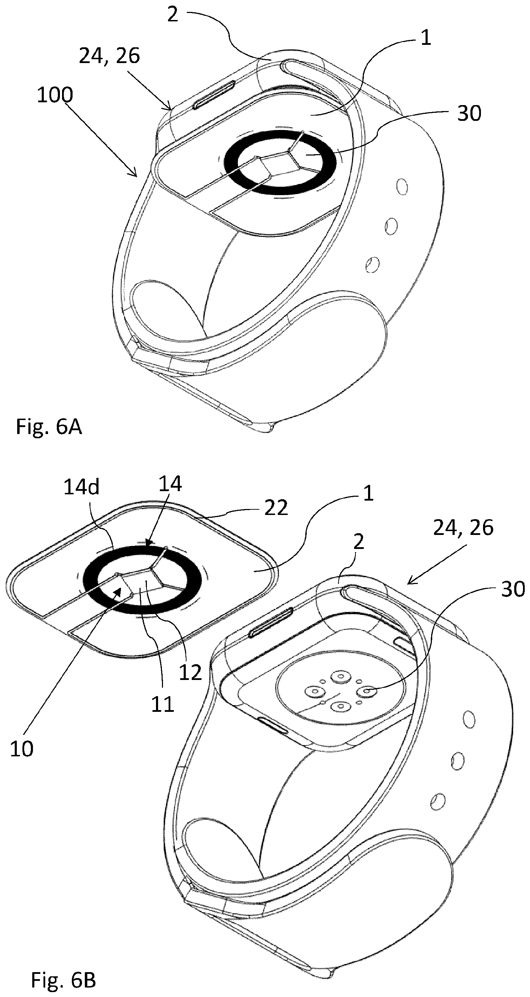

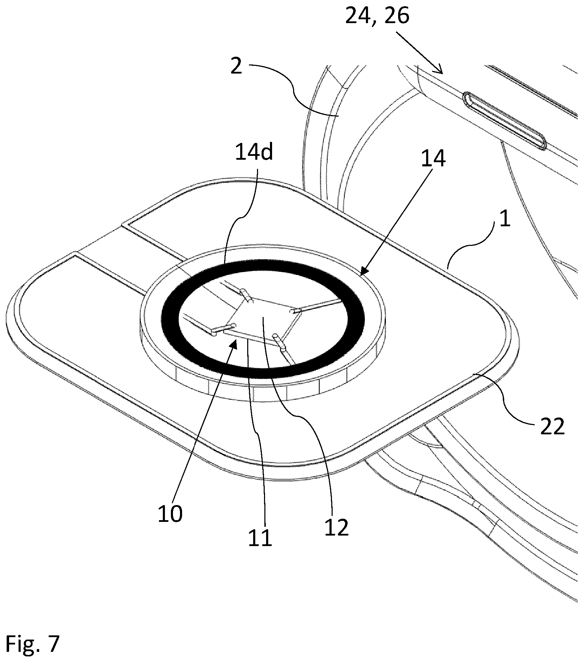

Furthermore, Figs. 6A-B show in conjunction with Fig. 7 an embodiment of a sensor unit 1 according to the present invention as well as monitoring system 100 comprising the sensor unit 1 and a transceiver unit 2 being formed by wristwatch 2. The wristwatch has integrated therein a transceiver circuit 24 (e.g. BLE (Bluetooth Low Energy)) configured to communicate with a remote device, and a battery 26 configured to supply the transceiver circuit 24 (and particularly the sensor unit 1) with electrical power.

Particularly, the sensor unit 1 comprises a carrier 13 formed as a flexible patch, wherein the signal processing integrated circuit 12 (e.g. ASIC) and the sensor 10 are arranged on the flexible patch 13, that is preferably configured to be connected to a bottom side of the transceiver unit/wristwatch 2 (e.g. smartwatch). The signal processing integrated circuit 12 and the sensor 12 are electrically connected to one another in a non-detachable manner. Particularly, the wristwatch 2 comprises at least one optical sensor 30 (e.g. for heartrate measurement and/or for measuring peripheral capillary oxygen saturation, i.e., SpO2), wherein the flexible patch 13 is configured to not cover the at least one optical sensor 30 of the wristwatch 2 when the flexible patch 13 is connected to said bottom side of the wristwatch 2. To this end, the flexible patch 13 can comprise a corresponding transparent portion or an opening configured to be aligned with the at least one optical sensor 30.

The sensor 10 of the sensor unit 1 may communicate with the transceiver unit / wristwatch 2 via an antenna 22 (e.g. coil), particularly via NFC. Particularly, the digital signal(s) provided by the signal processing integrated circuit 12 can be transmitted to the wristwatch 2 via said antenna 22. Furthermore, the sensor unit 1 may be provided with electrical power via the antenna 22, too. Particularly, the antenna 22 can be Qi inductive charging coil. Alternatively, the sensor unit 1 may be connected to the wristwatch 2 via an electrical connector via which electrical power and data relating to the measured concentrations of the respective (e.g. first or second analyte) can be passed.

The sensor unit 1 / patch 13 may be connected to the wristwatch 2 by a releasable connection system 14 that can be based on one of: a releasable mechanical connector configured to releasably engage with a mechanical connector of the transceiver unit /wristwatch 2 to connect the sensor unit 1 to the transceiver unit / wristwatch 2, an adhesive contact surface e.g. provided on the patch 13 configured to contact the transceiver unit / wristwatch 2 to provide a releasable adhesive connection of the sensor unit 1 to the transceiver unit / wristwatch 2, or a permanent magnet 14d (as indicated in Fig. 7 as an example) and/or a ferromagnetic element configured to facilitate a connection of the sensor unit 1 to the transceiver unit / wristwatch 2 via a magnetic force.

Particularly, the sensor 10 can be arranged on the signal processing integrated circuit or may be integrated therein to minimize the footprint of the assembly comprising the sensor 10 and signal processing integrated circuit 12 to avoid interference with the optical sensor(s) of the wristwatch 2. Particularly, the probe 11 of the sensor 10 can be formed as a microneedle array 11.

## Claims

1. A sensor unit (1), comprising:
- a sensor (10) comprising a probe (11) configured to be placed under the skin to contact interstitial fluid and to measure a concentration of a first analyte in the interstitial fluid, wherein the sensor (10) is configured to provide an analog measurement signal indicative of the concentration of the first analyte,
- a signal processing integrated circuit (12) permanently electrically connected to the sensor (10) in a non-detachable manner, wherein the signal processing integrated circuit (12) is configured to receive the analog measurement signal from the sensor (10), and
- a carrier (13) carrying the sensor (10) and the signal processing integrated circuit (12).

2. The sensor unit according to claim 1, further comprising a releasable connection system (14) configured to releasably connect the sensor unit (1) to a transceiver unit (2),

3. The sensor unit according to claim 1 or 2, wherein the signal processing integrated circuit (12) is configured to amplify the analog measurement signal and/or to convert the analog measurement signal into a digital signal being indicative of the concentration of the first analyte.

4. The sensor unit (1) according to one of the preceding claims, wherein the sensor unit (1) is a disposable sensor unit (1) that is configured to be discarded after a predefined time span, wherein said time span is smaller than or equal to 4 weeks, particularly smaller than or equal to three weeks, particularly smaller or equal to two weeks.

5. The sensor unit (1) according to one of the preceding claims, wherein the sensor unit (1) comprises a substrate (16), wherein particularly the substrate is a flexible substrate, wherein the signal processing integrated circuit (12) is arranged on the substrate (16).

6. The sensor unit (1) according to claim 5, wherein the sensor (10) is one of:
- connected to the substrate (16),
- integrated into the signal processing integrated circuit (12) or arranged thereon
- arranged on the carrier (13).

7. The sensor unit (1) according to claim 5 or 6, wherein the probe (11) comprises an elongated filament configured to be placed under the skin to contact interstitial fluid, wherein the elongated filament is connected to the substrate or forms a portion of the substrate.

8. The sensor unit (1) according to one of the claims 1 to 6, wherein the probe (11) comprises a microneedle array.

9. The sensor unit (1) according to one of the preceding claims, wherein the signal processing integrated circuit (12) is configured to calibrate the digital signal based on calibration data stored on the signal processing integrated circuit (12).

10. The sensor unit (1) according to one of the preceding claims, wherein the sensor unit (1) comprises an electrical connector (29) configured to make an electrical connection to a corresponding electrical connector of the transceiver unit (2) to provide electrical power to the sensor unit (1) and/or to pass the analog measurement signal or the digital signal to the transceiver unit (2), and particularly to receive digital data from the transceiver unit (2).

11. The sensor unit (1) according to one of the preceding claims, wherein the sensor unit (1) comprises an antenna (22), particularly a coil, configured for communication, particularly near field communication (NFC), with a corresponding antenna comprised by the transceiver unit (2) to provide electrical power to the sensor unit (1) and/or to transmit the digital signal to the transceiver unit (2), and particularly to receive digital data from the transceiver unit (2).

12. The sensor unit (1) according to one of the preceding claims, wherein the releasable connection system (14) comprises one of:
- a releasable mechanical connector (14a) configured to releasably engage with a mechanical connector (14b) of the transceiver unit (2) to connect the sensor unit (1) to the transceiver unit (2),
- an adhesive contact surface configured to contact the transceiver, providing a releasable adhesive connection of the sensor unit (1) to the transceiver unit (2),
- a permanent magnet (14d) and/or a ferromagnetic element (14d) to facilitate a connection to the transceiver unit (2) via a magnetic force.

13. The sensor unit (1) according to one of the preceding claims, wherein the carrier (13) forms a housing accommodating the signal processing integrated circuit (12), wherein the probe (11) protrudes from the housing, or wherein the carrier (13) is a flexible patch, wherein the signal processing integrated circuit (12) and the sensor (10) are arranged on the flexible patch, wherein particularly the flexible patch is configured to be connected to a bottom side of a transceiver unit (2) in form of a wristwatch, wherein particularly the wristwatch comprises at least one optical sensor, wherein the flexible patch is configured to not cover the at least one optical sensor of the wristwatch when the flexible patch is connected to said bottom side.

14. A monitoring system comprising a sensor unit (1) according to one of the preceding claims and a transceiver unit (2), wherein particularly the transceiver unit (2) comprises a transceiver circuit (24) configured to communicate with a remote device, and a battery (26) configured to supply the transceiver circuit (24) with electrical power.

15. The monitoring system according to claim 14, wherein the transceiver unit (2) is a wristwatch, wherein particularly the wristwatch has integrated therein the transceiver circuit (24) and accommodates the battery (26), and/or wherein the transceiver unit (2) comprises an electrical connector (21) configured to make an electrical connection to the electrical connector of the sensor unit (1) to provide electrical power to the sensor unit (1) and/or to receive the digital signal from the sensor unit (1), and particularly to pass digital data to the sensor unit (1), and/or wherein the transceiver unit (2) comprises an antenna (22), particularly a coil (22), configured for communication, particularly near field communication (NFC), with an antenna of the sensor unit (1) to provide electrical power to the sensor unit (1) and/or to receive the digital signal from the sensor unit (1), and particularly to transmit digital data to the sensor unit (1).
